(19) Europäisches Patentamt
European Patent Office
Office européen des brevets

(11) Numéro de publication : **0 620 221 A1**

# (12) DEMANDE DE BREVET EUROPEEN

(21) Numéro de dépôt : **94400820.0**

(22) Date de dépôt : **14.04.94**

(51) Int. Cl.[5] : **C07D 417/12**, C07D 417/14, C07D 471/06, A61K 31/40, A61K 31/47, A61K 31/41

(30) Priorité : **16.04.93 FR 9304535**

(43) Date de publication de la demande : **19.10.94 Bulletin 94/42**

(84) Etats contractants désignés : **AT BE CH DE DK ES FR GB GR IE IT LI LU MC NL PT SE**

(71) Demandeur : **ELF SANOFI**
**32-34, rue Marbeuf**
**F-75008 Paris (FR)**

(72) Inventeur : **Frehel, Daniel, 100 Allée de Barcelone**
**Apt. 206,**
**Résidence l'Antan**
**F-31000 Toulouse (FR)**
Inventeur : **Gully, Danièle**
**82 Route de Roquettes**
**F-31600 Saubens (FR)**
Inventeur : **Boigegrain, Robert**
**Chemin de Péret**
**F-34820 Assas (FR)**
Inventeur : **Badorc, Alain**
**1 Rue La Canal**
**F-31120 Roquettes (FR)**
Inventeur : **Bras, Jean-Pierre**
**16 Avenue de Castres**
**F-31500 Toulouse (FR)**
Inventeur : **Despeyroux, Pierre**
**190 Avenue du Comminges**
**F-31860 Labarthe S/Leze (FR)**

(74) Mandataire : **Polus, Camille et al**
**c/o Cabinet Lavoix**
**2, Place d'Estienne d'Orves**
**F-75441 Paris Cedex 09 (FR)**

(54) **5-Acylamino 1,2,4-thiadiazoles comme antagonistes ou agonistes de la cholécystokinine.**

(57) L'invention concerne les dérivés du thiadiazole répondant à la formule générale

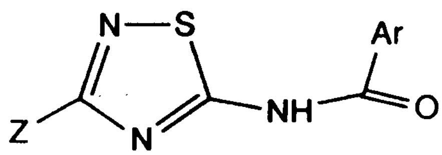

I

dans laquelle Ar représente un hétérocycle aromatique azoté, notamment indolyle substitué ou non sur l'azote par $CO-(C_1-C_4)$alkyle ; par $(CH_2)_nCOR$ dans lequel n représente 1 ou 2 et R représente $OR_1$ ou $NR_1R_2$ avec $R_1$ et $R_2$ identiques ou non représentant H ou $(C_1-C_4)$alkyle ; par hydroxyalkyle en $C_1$ à $C_4$ ; par alcoxyalkyle en $C_2$ à $C_6$ ; tétrahydropyranyle ; ou par une chaîne $-(CH_2)_3-$, dont le dernier carbone est fixé sur le noyau phényle de l'indole pour former un cycle à 6 sommets ;
Z représente :
(a) le groupe

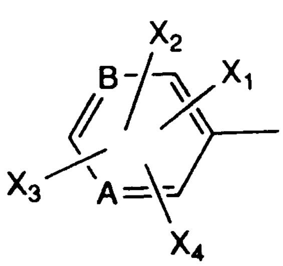

où A et B indépendamment l'un de l'autre représentent C, CH ou N ; et $X_1$, $X_2$, $X_3$, $X_4$ identiques ou non représentent H, $(C_1-C_3)$alkyle, $(C_1-C_3)$alcoxy, Cl, Br ou trifluorométhyle, ou bien
(b) un groupe naphtyle éventuellement substitué,
ainsi que leurs sels pharmaceutiquement acceptables.

EP 0 620 221 A1

La présente invention concerne des 5-acylamino-1,2,4-thiadiazoles, substitués en 3 par un groupe aromatique et liés par la fonction carbonyle à un hétérocycle aromatique azoté; ces composés ont une affinité pour les récepteurs biologiques de la cholecystokinine.

La cholecystokinine (CCK) est une hormone polypeptidique dont on trouve in vivo plusieurs fragments de 4 à 39 aminoacides. Ceux-ci ont de nombreuses activités physiologiques, notamment sur les voies biliaires, le tractus gastrointestinal ou dans le système nerveux central et périphérique, comme le décrit J.E. Morley dans Life Sciences 30 479-493 (1982).

On a mis en évidence 2 types de récepteurs, A et B, et l'existence d'autres types ou sous-types n'est pas exclue. Des agonistes et des antagonistes de l'action de la cholecystokinine sur ces récepteurs sont connus; on peut citer les dérivés de 3-amino-benzodiazépinones de J. Med. Chem. 32 13-16 (1989) ou les dérivés 2-acylaminothiazoles de EP-A-432 040 et EP-A-518 731, qui selon la nature des substitutions sont plus ou moins sélectifs des récepteurs de type A ou B.

Divers antagonistes et agonistes de la CCK sont actuellement étudiés en clinique humaine, notamment comme régulateurs de l'appétit, pour le traitement des désordres gastrointestinaux, pour lutter contre la douleur, pour diminuer l'anxiété, dans la schizophrénie ou pour supprimer les syndromes d'abstinence des pharmacodépendants.

Les composés de l'invention qui ont selon leur structure une activité agoniste ou antagoniste de la cholecystokinine sur ses récepteurs de type A ou B, répondent à la formule

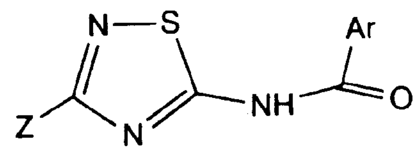

I

dans laquelle Ar représente un hétérocycle aromatique azoté choisi parmi quinolinyle, isoquinolinyle, benzimidazolyle et indolyle, ce dernier étant éventuellement substitué sur l'azote par un groupe W où W est choisi parmi

(i) -CO-($C_1$-$C_4$)alkyle ;

(ii) -$(CH_2)_n$COR, où R représente $OR_1$ ou $NR_1R_2$, $R_1$ et $R_2$ identiques ou non étant choisis parmi H et ($C_1$-$C_4$)alkyle et n est choisi parmi 1 et 2 ;

(iii) hydroxy($C_1$-$C_4$)alkyle ;

(iv) alcoxyalkyle en $C_2$-$C_6$ ;

(v) tétrahydropyranyle ;

(vi) une chaîne -$(CH_2)_3$-

dont le dernier carbone est fixé sur le noyau phényle de l'indole pour former un hétérocycle à 6 sommets ;

Z représente

(a) le groupe

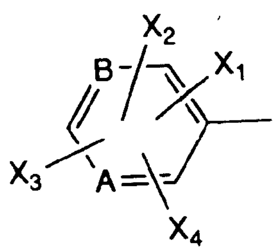

où A et B indépendamment l'un de l'autre représentent C, CH ou N ; et

$X_1$, $X_2$, $X_3$, $X_4$ identiques ou différents représentent un atome d'hydrogène, un groupe ($C_1$-$C_3$)alkyle, ($C_1$-$C_3$)alcoxy, halogéno, en particulier Cl ou Br ou trifluorométhyle ; ou bien

(b) un groupe naphtyle éventuellement substitué par un groupe ($C_1$-$C_3$)alkyle, ($C_1$-$C_3$)alcoxy ou un atome d'halogène.

L'invention concerne également les sels pharmaceutiquement acceptables des composés de formule I.

Parmi ces composés, on préfère ceux dans lesquels au moins 3 des substituants $X_1$ à $X_4$ représentent un atome d'hydrogène ou un groupe ($C_1$-$C_2$) alkyle ou ($C_1$-$C_2$) alcoxy et en particulier ceux choisis parmi les groupes méthyle ou méthoxy en position 2, 4, 6 du noyau aromatique ; par ailleurs, on préfère que Ar représente indolyle, substitué ou non, et plus particulièrement un groupe 2-indolyle, substitué ou non.

Les composés de formule I peuvent être préparés par condensation d'un aminothiadiazole de formule II

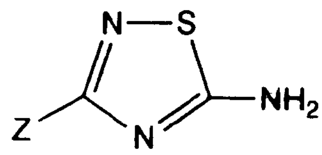

II

avec un acide ou un dérivé réactif d'un acide de formule Ar'COOH dans lequel Ar' représente Ar ou un dérivé de Ar dans lequel les fonctions sensibles aux conditions d'acylation habituelles auront été protégées. Parmi les dérivés d'acides convenables, on peut citer les chlorures et les anhydrides d'acide, éventuellement mixtes, ou les esters activés couramment utilisés en synthèse peptidique.

Certains composés de formule II sont connus; on peut se référer par exemple à EP-A-455 356, ou bien à DE 842 346 ou DE 955 684, où on décrit leur préparation par action d'un thiocyanate alcalin sur la N-halogénoamidine correspondante. D'autres composés de formule II sont nouveaux et sont préparés selon des méthodes connues. Ils peuvent par exemple être obtenus par action de l'ammoniac liquide sur le 5-chlorothiadiazole, convenablement substitué en 3, obtenu par action du chlorure de trichlorométhane sulfényle sur l'amidine convenablement substituée. Les amidines sont obtenues en appliquant des méthodes connues à partir des nitriles correspondants de formule III

$$Z\text{-}C{\equiv}N \qquad III$$

soit par l'intermédiaire de l'oxime qui peut être réduite en amidine par l'hydrogène en présence d'un catalyseur soit par l'intermédiaire de l'iminoester qui est traité par $NH_4Cl$.

Les conditions de toutes ces réactions sont bien connues de l'homme du métier.

Lorsque les nitriles ne sont pas commerciaux, on peut les préparer par action du réactif de Lawesson sur l'amide primaire correspondante, obtenue à partir de l'acide carboxylique.

Pour la préparation des acides Ar-COOH et leurs dérivés, et la méthode de condensation sur l'amine on peut se référer à EP-A-432 040, et à DE-3 907 390.

Les composés de l'invention déplacent la cholécystokinine iodée ou ses fragments biologiques iodés, de ses récepteurs de type A ou B.

L'affinité pour les récepteurs de type A a été étudiée, in vitro, sur un homogénat de pancréas de rat par rapport à la CCK 8 S iodée selon la méthode décrite notamment dans Endocrinology 109, 1746 (1981); dans ces conditions, les produits décrits dans les exemples suivants ont des $CI_{50}$ comprises entre $10^{-8}$ M et $10^{-10}$ M.

L'affinité pour les récepteurs de type B a été étudiée par la méthode décrite dans J. Neurochem. 37, 443 (1981) ; sur un homogénat de cortex de cobaye, les composés des exemples ont des $CI_{50}$ de l'ordre de $10^{-7}$ M.

Pour déterminer si les composés sont des agonistes ou des antagonistes sur les récepteurs de type A, l'homme du métier sait qu'il peut étudier l'action des composés sur la secrétion d'amylase par des acini pancréatiques de rats, selon une méthode décrite notamment dans J. Biol. Chem. 254 (12) 5321-5327 (1979); dans ces conditions, un agoniste stimule la secrétion d'amylase, tandis qu'un antagoniste diminue la secrétion induite par le fragment CCK 8 S. Parmi les composés de formule I, ceux pour lesquels $X_1$, situé en position 2, est $OCH_3$ et $X_2$ et $X_3$ sont indépendamment l'un de l'autre méthyle ou méthoxy sont des composés agonistes préférés.

Par ailleurs l'activité antagoniste sur les récepteurs de type B, pourra être étudiée par la méthode décrite dans European J. of Pharmacology 232, 13-19 (1993).

En raison de leur aptitude à déplacer la cholécystokinine de ses récepteurs A ou B, les composés de l'invention peuvent être utilisés avantageusement pour le traitement ou la prévention des maladies dans lesquelles la cholecystokinine ou ses fragments sont impliqués.

Lorsque ces composés sont des antagonistes de la CCK, ils sont utilisés contre les ulcères, les cancers du pancréas et de la rate, les pancréatites, l'hyperinsulinémie, le syndrome du colon irritable ou encore dans les psychoses, l'anxiété, la maladie de Parkinson, pour diminuer les dyskinésies tardives, comme régulateurs de l'appétit ou dans le traitement des douleurs ou pour lutter contre le syndrome d'abstinence chez les pharmacodépendants.

Lorsque ces composés sont des agonistes de la CCK sur les récepteurs de type A, ils sont utilisés comme anorexigènes ou encore dans le traitement des psychoses, pour améliorer la mémoire ou comme antichoc.

Les compositions pharmaceutiques qui contiennent comme principe actif au moins un composé de formule I ou l'un de ses sels pharmaceutiquement acceptables sont un autre objet de l'invention.

Dans les compositions pharmaceutiques de la présente invention pour l'administration orale, sublinguale,

sous-cutanée, intramusculaire, intraveineuse, topique, intratrachéale, intranasale, transdermique ou rectale, les principes actifs de formule I ci-dessus, ou leurs sels éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades ou lotions.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues.

On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté des produits de formule I ci-dessus ou d'un de leurs sels pharmaceutiquement acceptables, d'autres principes actifs qui peuvent être utiles dans le traitement des troubles ou maladies indiquées ci-dessus.

Les doses administrées dépendent de la nature et de l'importance de la maladie,du composé et de la voie d'administration. Elles seront généralement comprises entre 20 et 100 mg par jour chez l'homme adulte par voie orale et 3 à 10 mg en injection.

Dans ce qui suit, on décrit des exemples de préparation des exemples des composés de l'invention.

## EXEMPLE 1

Acide 2-[3-(2-chlorophényl)-1,2,4,-thiadiazolyl-5-amino-carbonyl]indolyl-1-acétique et son ester méthylique.

Formule I : Z = 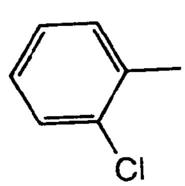

Ar = 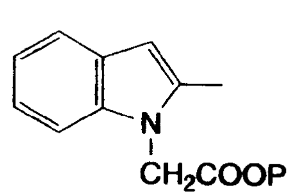 où P est H ou $CH_3$

a) 2-chlorobenzamidine

On ajoute 200 ml de méthanol saturé en gaz chlorhydrique, 56g de 2-chlorobenzonitrile à une température voisine de 0°C. L'ensemble est maintenu à + 5°C pendant une nuit (au réfrigérateur). Le milieu réactionnel est ensuite évaporé, sans chauffer. le résidu est repris par 200 ml de méthanol sec. La solution est refroidie à une température voisine de 0°C et du gaz d'ammoniac y est introduit jusqu'à pH basique. On chauffe le milieu réactionnel pendant 3 heures. Après évaporation à sec, la 2-chlorobenzamidine est purifiée par chromatographie sur colonne de silice (éluant : dichlorométhane/méthanol-8/2).
F = 236°C (bromhydrate) - Rendement : 70%.

b) 5-chloro-3-(2-chlorophényl)-1,2,4-thiadiazole

A un mélange de 37 g de bromhydrate de 2-chlorobenzamidine dans 160 ml de dichlorométhane refroidi à - 10°C, on ajoute goutte à goutte 36 g de chlorure de trichlorométhanesulfényle dissout dans 160 ml de dichlorométhane. Après 30 mn d'agitation on introduit goutte à goutte dans le milieu, toujours à - 10°C, 65 ml d'une solution aqueuse de NaOH à 50%. On laisse la solution revenir à température ambiante et on agite pendant 2 h. On ajoute de l'eau au milieu réactionnel et on décante. La phase organique est lavée à l'eau, séchée sur sulfate de sodium et évaporée à sec.

c) 5-amino-3-(2-chlorophényl)-1,2,4-thiadiazole

Dans un autoclave, on met en suspension les cristaux obtenus à l'étape précédente dans 100 ml de méthanol et on ajoute en refroidissant un large excès d'ammoniac liquide. On laisse revenir à température ambiante et on agite 24 h avant de concentrer à sec. Le produit final est purifié par chromatographie sur colonne de silice (Eluant : dichlorométhane/hexane - 80/20). F = 136°C
Rendement global des étapes b et c : 61%.

d) 2-[3-(2-chlorophényl)-1,2,4-thiadiazolyl-5-aminocarbonyl]indolyl-1-acétate de méthyle.

On dissout 3,6 ml de pyridine dans 40 ml de dichlorométhane et on ajoute 0,90 ml de chlorure de thionyle à une température voisine de - 5°C. On laisse 30 mn à - 5°C et on ajoute par portions 3 g d'acide 1-(méthoxycarbonylméthyl)-indolyl-2-carboxylique. On agite 30 mn à la même température puis on ajoute par portions 2,4 g de 5-amino-3-(2-chlorophényl)-1,2,4,-thiadiazole. On laisse revenir à température ambiante et on agite 18 heures. On lave le milieu réactionnel à l'eau, on décante la solution, on sèche la phase organique sur sulfate de sodium et on évapore à sec. Le produit est purifié par chromatographie sur colonne de silice (éluant : dichlorométhane/méthanol - 95/5) puis recristallisé dans l'éther isopropylique.
F = 197 °C - Rendement 69%.

e) Acide 2-[3-(2-chlorophényl)-1,2,4-thiadiazolyl-5-aminocarbonyl]-indolyl-1-acétique

On met 1 g de 2-[3-(2-chlorophényl)-1,2,4-thiadiazolyl-5-aminocarbonyl]indolyl-1-acétate de méthyle en suspension dans 20 ml de méthanol et on ajoute à température ambiante 7 ml de solution aqueuse de NaOH 1N. On agite l'ensemble à température ambiante pendant 3 heures ; le milieu réactionnel est concentré ; on y ajoute de l'eau et on ajuste le pH à 3 par addition de $KHSO_4$. On isole le précipité ;
F = 255°C - Rendement : 92%.

## EXEMPLE 2

Acide 2-[3-(2,4,6-triméthoxyphényl)-1,2,4,-thiadiazolyl-5-aminocarbonyl]indolyl-1-acétique et son ester méthylique.

Formule I : Z = 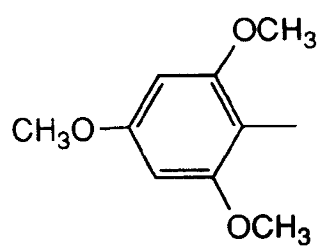

Ar = 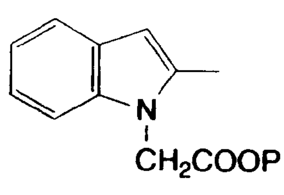 où P = H ou $CH_3$

a) 2,4,6-triméthoxybenzamidoxime

A une suspension de 13,8 g de chlorhydrate d'hydroxylamine dans 100 ml d'éthanol, on ajoute à une température voisine de 15°C une solution de $C_2H_5ONa$ préparée en dissolvant 4,3 g de sodium dans 100 ml d'éthanol. On introduit dans le milieu 12 g de 2,4,6-triméthoxybenzonitrile, puis on chauffe à reflux pendant 41 h avant d'évaporer à sec. On lave les cristaux avec de l'eau et du dichlorométhane. F = 205°C; Rendement : 71%.

b) 2,4,6-triméthoxybenzamidine

Dans un autoclave, on dissout 3,4 g de 2,4,6-triméthoxybenzamidoxime dans 120 ml d'un mélange méthanol/dichlorométhane/acide acétique (2/2/1;(V/V/V)), et on hydrogène sous une pression de $2.10^6$Pa en présence de 1 g de nickel de Raney. Après 2 heures d'hydrogénation, on sépare le catalyseur et on concentre à sec.

c) 5-chloro-3-(2,4,6-triméthoxyphényl)-1,2,4-thiadiazole

On dissout la résine obtenue à l'étape précédente dans 20 ml de dichlorométhane refroidi à -10°C et on ajoute goutte à goutte, 2,8 ml de chlorure de trichlorométhanesulfényle dissout dans 20 ml de dichlorométhane. On agite 30 minutes le milieu réactionnel à -10°C et on ajoute goutte à goutte 5 ml de solution aqueuse de NaOH à 30% et on agite ensuite 2 heures à température ambiante avant d'ajouter de l'eau au milieu réactionnel; après décantation la phase organique est lavée à l'eau, et séchée sur sulfate de sodium et évaporée à sec.

d) 5-amino-3-(2,4,6-triméthoxyphényl)-1,2,4-thiadiazole

Dans un autoclave, on met en suspension les cristaux obtenus à l'étape précédente dans 100 ml de méthanol et on ajoute en refroidissant un gros excès d'ammoniac liquide. On laisse remonter à température ambiante et on agite 18 heures avant d'évaporer à sec. On ajoute sur le résidu 100 ml de solution aqueuse d'acide chlorhydrique 2 N; le précipité formé est filtré, lavé à l'eau puis à l'acétone.

F = 215°C (chlorhydrate); Rendement des 3 étapes : 41%.

e) 2-[3-(2,4,6-triméthoxyphényl)-1,2,4,-thiadiazolyl-5-aminocarbonyl]indolyl-1-acétate de méthyle

On dissout 1,5 ml de pyridine dans 20 ml de dichlorométhane et on ajoute 0,34 ml de chlorure de thionyle à une température voisine de -5°C. On laisse 30 minutes à -5°C et on ajoute par portions, 1 g d'acide 1-(méthoxycarbonylméthyl)-indolyl-2 carboxylique. On agite l'ensemble 30 minutes à la même température puis on ajoute par portions, 1,24 g de chlorhydrate de 5-amino-3-(2,4,6-triméthoxyphényl)-1,2,4-thiadiazole; on laisse revenir à température ambiante et on agite le mélange 18 heures. On lave le milieu réactionnel à l'eau. Après décantation, on sèche la phase organique sur sulfate de sodium et on évapore à sec. Le produit final est purifié par chromatographie sur colonne de silice (éluant : dichlorométhane/méthanol-95/5) et on cristallise dans l'éther isopropylique.

F = 205°C - Rendement : 78%.

f) Acide 2-[3-(2,4,6-triméthoxyphényl)-1,2,4,-thiadiazolyl-5-aminocarbonyl]indolyl-1-acétique

On met en suspension 0,7 g de 2-[3-(2,4,6-triméthoxyphényl)-1,2,4-thiadiazolyl-5-aminocarbonyl]indolyl-l-acétate de méthyle dans 15 ml de méthanol et on ajoute à température ambiante 4,4 ml de solution aqueuse de NaOH 1 N. On agite la solution à température ambiante 3 heures. Le milieu réactionnel est évaporé. On ajoute de l'eau et on amène le pH à 3 par addition de $KHSO_4$. Le précipité formé est isolé.

F = 260°C ; Rendement : 65%.

## EXEMPLE 3

Acide 2-[3-(2,6-diméthoxy-4-méthylphényl)-1,2,4,thiadiazolyl-5-aminocarbonyl]indolyl-1-acétique et son ester méthylique.

Formule I : Z = 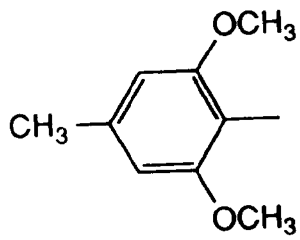

Ar = 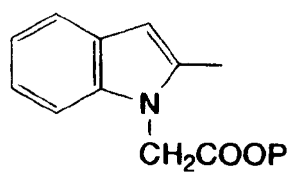 ou P = H ou $CH_3$

a) Acide 2,6-diméthoxy-4-méthyl benzoïque

A 200 ml de tétrahydrofurane, on ajoute 104 ml de solution de n-butyl lithium 1,6 M dans l'hexane, puis, goutte à goutte 25 ml de 3,5-diméthoxy toluène à une température comprise entre 0° et 5°C. On agite à 5°C pendant 1 h 30 puis on introduit en 30 minutes dans le milieu réactionnel un excès de $CO_2$ gazeux. L'ensemble est introduit dans 200 ml de solution aqueuse de HCl 0,5 N et la phase aqueuse est extraite par de l'acétate d'éthyle. Le résidu est concentré et chromatographié sur colonne de silice (éluant : dichlorométhane/méthanol - 93/7).

F = 178°C; Rendement : 72%.

b) 2,6-diméthoxy-4-méthyl benzamide

On dissout 7 g de l'acide précédent dans 100 ml de dichlorométhane et on ajoute goutte à goutte à la solution, 6,7 ml de chlorure d'oxalyle, en maintenant la température à 10°C. On laisse le mélange 4 heures à température ambiante, avant d'évaporer à sec; on verse sur le résidu, 100 ml d'ammoniac liquide et on laisse l'ensemble à 20°C pendant 18 heures en autoclave. Après évaporation à sec, on verse sur le résidu un mélange d'eau et d'acétate d'éthyle et on extrait le produit final dans la phase organique. Le produit est purifié par chromatographie sur colonne de silice (éluant : dichlorométhane/méthanol-9/1).

F = 201°C; Rendement : 73%.

c) 2,6-diméthoxy-4-méthylbenzonitrile

On met en suspension 3 g d'amide dans 60 ml de toluène et on ajoute 3,7 g de réactif de Lawesson avant de chauffer le mélange à 90°C pendant 1 h 30. On évapore le milieu réactionnel à sec et on reprend le résidu par de l'acétate d'éthyle; la solution organique est lavée avec une solution aqueuse de NaOH, séchée et concentrée. Le résidu est purifié par chromatographie sur colonne de silice (éluant : dichlorométhane/toluène - 1/1).

F = 122°C; Rendement : 92%.

d) 2,6-diméthoxy-4-méthylbenzamidoxime

A une solution de 2,6 g du benzonitrile précédent dans 30 ml d'éthanol, on ajoute 2,2 g de chlorhydrate d'hydroxylamine puis 1,34 g de NaOH en pastilles. On porte au reflux pendant 48 heures et on évapore à sec le milieu réactionnel. On reprend par 100 ml d'une solution aqueuse de HCl 1 N et on lave avec de l'acétate d'éthyle; par addition d'une solution aqueuse de NaOH 1 N, la solution est amenée à pH 5 et puis on extrait le chlorhydrate de l'amidoxime dans l'acétate d'éthyle.

F = 140°C; Rendement : 75%.

e) 5-amino-3-(2,6-diméthoxy-4-méthylphényl)-1,2,4-thiadiazole

Dans un autoclave, on introduit 3,2 g de 2,6-diméthoxy-4-méthyl benzamidoxime en solution dans 50 ml de méthanol, et on ajoute 400 mg de Ni de Raney; on hydrogène à 20°C sous une pression de $1,4.10^6$Pa d'hydrogène pendant 12 heures. On filtre le catalyseur sur un lit de talc et on évapore à sec le filtrat méthanolique. On dissout l'huile jaune résiduelle dans 50 ml de dichlorométhane et on ajoute à 20°C, 2,84 g de chlorure de trichlorométhanesulfényle,puis, goutte à goutte à -10°C, 20 ml d'une solution de 3 g de NaOH dans 20 ml d'eau. On laisse revenir à 20°C en fin d'addition et on agite le mélange pendant 3 heures. On extrait alors du milieu réactionnel le produit souhaité avec du dichlorométhane. On dissout cette huile dans un mélange de 40 ml de méthanol et de 10 ml de dichlorométhane et on l'introduit, après

refroidissement, dans un autoclave contenant 200 ml d'ammoniac liquide; après 12 heures à température ambiante, on concentre le résidu et on le reprend par du $CH_2Cl_2$; on lave la phase organique à l'eau, on la concentre et on purifie le produit final par chromatographie sur colonne de silice (éluant : dichlorométhane/méthanol - 96/4).

F = 117°C - Rendement global : 58%.

f) 2-[3-(2,6-diméthoxy-4-méthylphényl)-1,2,4,-thiadiazolyl-5-aminocarbonyl]indolyl-1-acétate de méthyle

On introduit 1 ml de pyridine dans 10 ml de dichlorométhane à 0°C puis 0,25 ml de chlorure de thionyle; après 30 mn on ajoute par fractions dans le milieu réactionnel 0,2 g de chlorure de l'acide 1-(méthoxycarbonylméthyl)indolyl-2-carboxylique puis goutte à goutte, 0,8 g de 5-amino-3-(2,6-diméthoxy-4-méthylphényl)-1,2,4-thiadiazole en solution dans 10 ml de dichlorométhane. On laisse le mélange 5 heures à 20°C avant d'y introduire un volume d'eau. On sépare le mélange de la phase organique, on la sèche et on évapore le solvant. On purifie l'huile jaune obtenue par chromatographie sur colonne de silice (éluant dichlorométhane/éther diéthylique -95/5). Le produit est recristallisé dans l'isopropanol.

F = 122°C; Rendement : 65%.

g) acide 2-[3-(2,6-diméthoxy-4-méthylphényl)-1,2,4,-thiadiazolyl-5-aminocarbonyl]indolyl-1-acétique

On introduit 0,35 g de l'ester méthylique précédent dans 5 ml de méthanol puis 1,5 ml de solution aqueuse de NaOH 1 N. Après 6 heures d'agitation on évapore le méthanol, on verse sur le résidu 50 ml d'eau, et on acidifie le milieu avec une solution aqueuse à 5% (poids/volume), de $KHSO_4$. On extrait la phase aqueuse au dichlorométhane et on concentre la phase organique. Le produit est obtenu sous forme de cristaux jaunes.

F = 235°C; Rendement : 82%.

## EXEMPLE 4

N-[3-(2,6-diméthoxy-4-méthylphényl)-1,2,4,-thiadiazol-5-yl]-1-(2-tétrahydropyranyl)-indolyl-2-carboxamide

Formule I : Z = 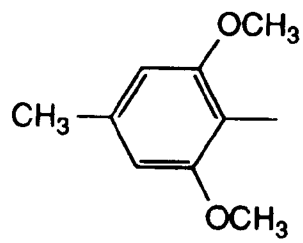

Ar = 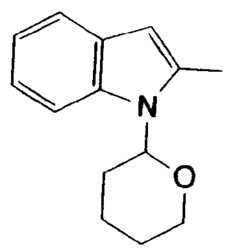

On introduit 1,8 ml de pyridine dans 20 ml de dichlorométhane. On porte le mélange à 0°C et on y ajoute 0,36 ml de chlorure de thionyle. Après 30 minutes, on introduit par fractions 1,2 g de chlorure d'acide 1-(2-tétrahydropyrannyl)-indolyl-2-carboxylique, puis, goutte à goutte, 1,2 g de 5-amino-3-(2,6-diméthoxy-4-méthylphényl)-1,2,4-thiadiazole en solution dans 10 ml de dichlorométhane. Après 3 heures à -20°C , on introduit un volume d'eau dans le milieu réactionnel et on extrait la phase aqueuse au dichlorométhane. Les phases organiques sont ensuite séchées et concentrées. Le résidu est purifié par chromatographie sur colonne de silice, (éluant : dichlorométhane/méthanol 99/1).

F = 142°C; Rendement = 80%

## EXEMPLE 5

N-[3-(2-chlorophényl)-1,2,4-thiadiazol-5-yl]-1-(2-tétrahydropyranyl)-indolyl-2-carboxamide

Formule I : Z = 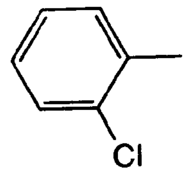

Ar = 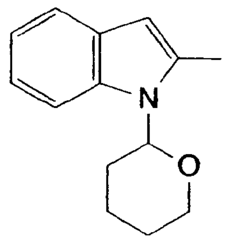

préparé comme à l'exemple 4 à partir du 5-amino-3-(2-chlorophényl)-1,2,4-thiadiazole qui fond à 136°C.
F = 182°C; Rendement : 90%.

**EXEMPLE 6**

N-[3-(2,6-diméthoxy-4-méthylphényl)-1,2,4-thiadiazol-5-yl]-indolyl-2-carboxamide

Formule I : Z = 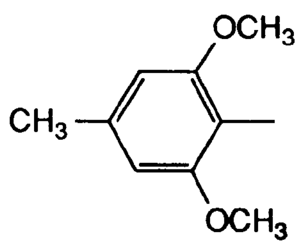 Ar = 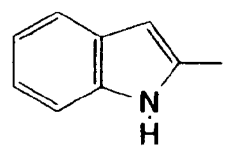

On dissout 0,4 g du composé de l'Exemple 4 dans 25 ml de méthanol et on ajoute 0,8 ml de solution aqueuse de HCl 6 N. On maintient à la température de reflux 6 heures; puis on évapore le solvant; le produit final est extrait en milieu aqueux dans le dichlorométhane.
F = 247°C - Rendement 94%.

**EXEMPLE 7**

N-[3-(2-chlorophényl)-1,2,4-thiadiazol-5-yl]-indolyl-2-carboxamide

Formule I : Z = 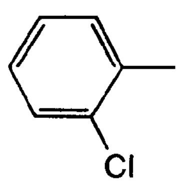 Ar = 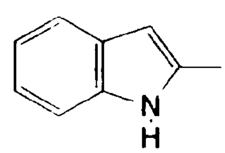

préparé comme à l'exemple 6 à partir du composé de l'Exemple 5.
F = 291°C - Rendement 86%.

**EXEMPLE 8**

N-[3-(2-chlorophényl)-1,2,4-thiadiazol-5-yl]-quinolyl-2-carboxamide

Formule I : Z = 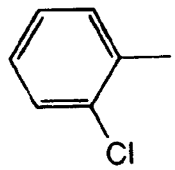 Ar = 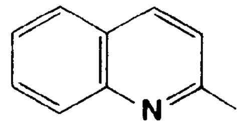

En utilisant le procédé de l'exemple 1 et à partir de l'acide carboxylique approprié, le composé du titre a été obtenu.
F = 214°C - Rendement 85%.

**EXEMPLE 9**

N-[3-(2-chlorophényl)-1,2,4-thiadiazol-5-yl][5,6-dihydro-([4H]-pyrrolo[3,2,1-ij]]quinolyl)]-2-carboxamide

Formule I : Z = 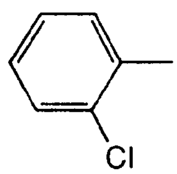 Ar = 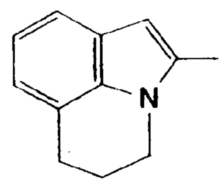

En utilisant le procédé de l'exemple 1 et à partir de l'acide carboxylique approprié, le composé du titre a été obtenu.
F = 195°C - Rendement 79%.

En procédant selon les exemples 1 à 9 précédents, on prépare les composés de formule I des exemples 10 à 21 répertoriés dans le Tableau I suivant. Les 5-amino-1,2,4-thiadiazoles intermédiaires P1 à P4 conduisant aux composés des exemples 10 à 21 sont rapportés dans le Tableau II suivant.

## TABLEAU I

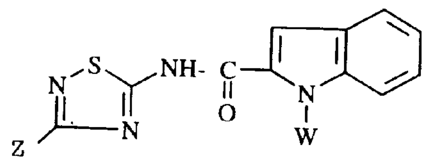

(I)

| Exemple numéro | Z | W | F ; °C |
|---|---|---|---|
| 10 | 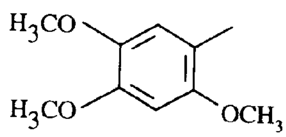 | 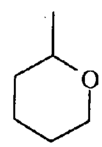 | 214 |
| 11 | 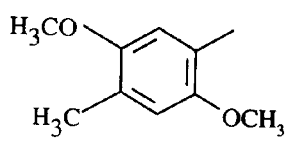 | 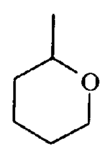 | 226 |
| 12 | 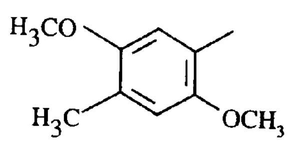 | $-CH_2COOCH_3$ | 212,5 |
| 13 | 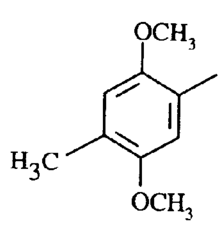 | $-CH_2COOH$ | 212 |

## TABLEAU I (suite 1)

| Exemple numéro | Z | W | F ; °C |
|---|---|---|---|
| 14 | 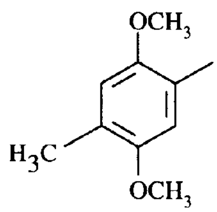 | H | 255 |
| 15 | 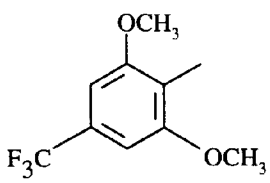 | $-CH_2COOCH_3$ | 262 |
| 16 | 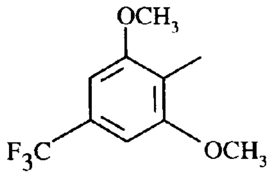 | H | 168 |
| 17 | 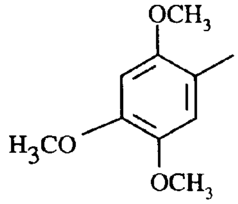 | $-CH_2COOCH_3$ | 178 |

**TABLEAU I (suite 2)**

| Exemple numéro | Z | W | F ; °C |
|---|---|---|---|
| 18 | 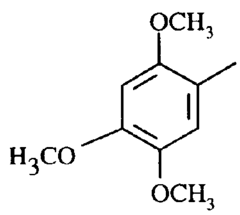 | $-CH_2COOH$ | 214 |
| 19 | 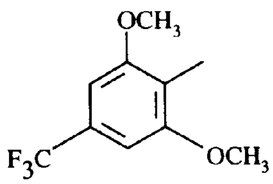 | 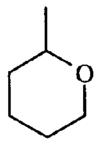 | 283 |
| 20 | 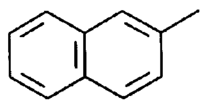 | $-CH_2COOCH_3$ | 236 |
| 21 | 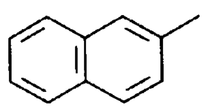 | $-CH_2COOH$ | 283 |

## TABLEAU II

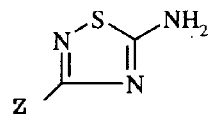

(II)

| Composé numéro | Z | F ; °C |
|---|---|---|
| P 1 | 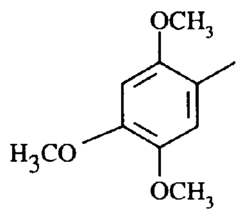 | 233 |
| P 2 | 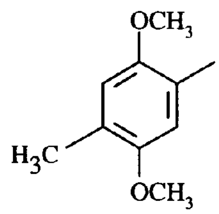 | 195 |
| P 3 | 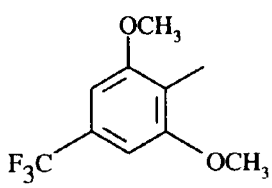 | 198 |
| P 4 | 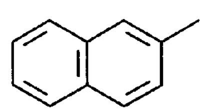 | 153 |

## Revendications

1. Composés de formule I

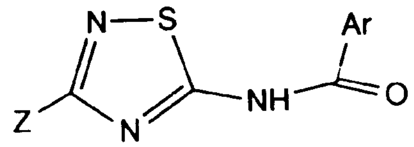

I

dans laquelle Ar représente un hétérocycle aromatique azoté choisi parmi quinolinyle, isoquinolinyle, benzimidazolyle et indolyle, ce dernier étant éventuellement substitué sur l'azote par un groupe W où W est choisi parmi

(i) -CO-($C_1$-$C_4$)alkyle ;

(ii) $-(CH_2)_nCOR$, où n représente 1 ou 2 et R représente $OR_1$ ou $NR_1R_2$, $R_1$ et $R_2$ identiques ou non étant choisis parmi H et ($C_1$-$C_4$)alkyle ;

(iii) hydroxy($C_1$-$C_4$)alkyle ;

(iv) alcoxyalkyle en $C_2$-$C_6$ ;

(v) tétrahydropyranyle ;

(vi) une chaîne $-(CH_2)_3-$

dont le dernier carbone est fixé sur le noyau phényle de l'indole pour former un hétérocycle à 6 sommets ;

Z représente

(a) le groupe

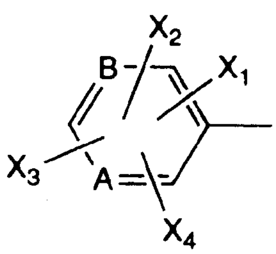

où A et B indépendamment l'un de l'autre représentent C, CH ou N ; et

$X_1$, $X_2$, $X_3$, $X_4$ identiques ou différents représentent un atome d'hydrogène, de chlore ou de brome, un groupe ($C_1$-$C_3$)alkyle, ($C_1$-$C_3$)alcoxy ou trifluorométhyle ;

(b) un groupe naphtyle éventuellement substitué par un groupe ($C_1$-$C_3$)alkyle, ($C_1$-$C_3$)alcoxy ou un atome d'halogène,

ainsi que leurs sels pharmaceutiquement acceptables.

2. Composés de formule I selon la revendication 1, dans laquelle au moins 3 des groupes $X_1$, $X_2$, $X_3$, $X_4$ identiques ou non représentent un atome d'hydrogène ou un groupe ($C_1$-$C_2$)alkyle ou ($C_1$-$C_2$)alcoxy.

3. Composés de formule I selon la revendication 1, dans laquelle les groupes $X_1$, $X_2$, $X_3$ sont situés en position 2, 4 et 6 du noyau aromatique et représentent méthyle ou méthoxy.

4. Composés de formule I selon l'une des revendications 1 à 3, dans laquelle Ar représente un groupe 2-indolyle, éventuellement substitué sur l'azote par un groupe W où W est choisi parmi

(i) -CO-($C_1$-$C_4$)alkyle ;

(ii) $-(CH_2)_nCOR$, où n représente 1 ou 2 et R représente $OR_1$ ou $NR_1R_2$, $R_1$ et $R_2$ identiques ou non étant choisis parmi H et ($C_1$-$C_4$)alkyle ;

(iii) hydroxy($C_1$-$C_4$)alkyle ;

(iv) alcoxyalkyle en $C_2$-$C_6$ ;

(v) tétrahydropyranyle ;

(vi) une chaîne $-(CH_2)_3-$
dont le dernier carbone est fixé sur le noyau phényle de l'indole pour former un hétérocycle à 6 sommets.

5. Composé de formule I selon la revendication 1, dans laquelle $X_1$, situé en position 2, représente méthoxy et $X_2$ et $X_3$ sont indépendamment l'un de l'autre méthyle ou méthoxy.

6. Procédé pour la préparation des composés selon l'une quelconque des revendications 1 à 5, qui consiste à faire réagir un dérivé 5-amino-thiadiazole de formule II

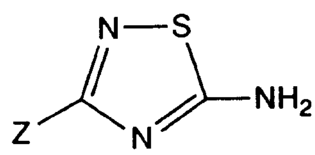

II

dans laquelle Z a la même signification que dans la revendication 1, sur un dérivé réactif d'un acide de formule Ar'COOH, où Ar' représente :
(i) Ar, Ar ayant la même signification que dans la revendication 1, ou
(ii) un dérivé de Ar dans lequel les fonctions sensibles aux conditions d'acylation ont été protégées,
puis à déprotéger lorsque nécessaire lesdites fonctions sensibles du noyau aromatique Ar.

7. Composition pharmaceutique contenant un composé selon l'une des revendications 1 à 5 en association avec un véhicule pharmaceutique.

8. Composés de formule I selon la revendication 1, dans laquelle Ar représente un hétérocycle aromatique azoté choisi parmi quinoléine, isoquinoléine, benzimidazole et indole, ledit groupe indole pouvant être éventuellement substitué sur l'azote par $CO-(C_1-C_4)$alkyle; par $CH_2COR$ dans lequel R représente $OR_1$ ou $NR_1R_2$ avec $R_1$ et $R_2$ identiques ou non représentant H ou $(C_1-C_4)$alkyle; par hydroxyalkyle en $C_1$ à $C_4$; par alcoxyalkyle en $C_2$ à $C_6$; tétrahydropyranyle; ou par une chaîne $-(CH_2)_3-$, dont le dernier carbone est fixé sur le noyau phényle de l'indole pour former un cycle à 6 sommets ;
Z représente le groupe

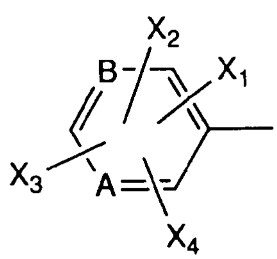

où A et B indépendamment l'un de l'autre représentent C, CH ou N ; et $X_1$, $X_2$, $X_3$, $X_4$ identiques ou non représentent H, $(C_1-C_3)$alkyle, $(C_1-C_3)$alcoxy, Cl, Br ou trifluorométhyle,
ainsi que leurs sels pharmaceutiquement acceptables.

Office européen des brevets

# RAPPORT DE RECHERCHE EUROPEENNE

Numero de la demande
EP 94 40 0820

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| D,A | EP-A-0 518 731 (ELF SANOFI) 16 Décembre 1992<br>* le document en entier * | 1-8 | C07D417/12<br>C07D417/14<br>C07D471/06<br>A61K31/40<br>A61K31/47<br>A61K31/41 |
| D,A | EP-A-0 432 040 (ELF SANOFI) 12 Juin 1991<br>* le document en entier * | 1-8 | |
| A | EP-A-0 455 356 (LILLY INDUSTRIES LIM.) 6 Novembre 1991<br>* page 6, ligne 20 - ligne 29 *<br>* revendication 1 * | 1-8 | |
| A | EP-A-0 343 894 (PFIZER) 29 Novembre 1989<br>* page 4, ligne 24 - ligne 35 *<br>* page 5, ex. 2 * | 1-8 | |
| | | | **DOMAINES TECHNIQUES RECHERCHES (Int.Cl.5)** |
| | | | C07D |

Le présent rapport a été établi pour toutes les revendications

| Lieu de la recherche | Date d'achèvement de la recherche | Examinateur |
|---|---|---|
| LA HAYE | 14 Juillet 1994 | Kissler, B |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)